Europäisches Patentamt

European Patent Office

Office européen des brevets

(19)

(11) Veröffentlichungsnummer: **0 162 178**
**B1**

(12) **EUROPÄISCHE PATENTSCHRIFT**

(45) Veröffentlichungstag der Patentschrift:
02.08.89

(51) Int. Cl.⁴: **A 61 N 1/05**

(21) Anmeldenummer: **84810312.3**

(22) Anmeldetag: **25.06.84**

(54) **Leitvorrichtung, insbesondere zum mindestens teilweisen Einsetzen in einen menschlichen oder tierischen Körper, mit einer zumindest aus einem Leiter gebildeten Wendel.**

(30) Priorität: **09.04.84 CH 1773/84**

(43) Veröffentlichungstag der Anmeldung:
**27.11.85 Patentblatt 85/48**

(45) Bekanntmachung des Hinweises auf die Patenterteilung:
**02.08.89 Patentblatt 89/31**

(84) Benannte Vertragsstaaten:
**DE FR GB IT NL SE**

(56) Entgegenhaltungen:
**US-A-3 333 045**

(73) Patentinhaber: **INSTITUT STRAUMANN AG, CH-4437 Waldenburg (CH)**

(72) Erfinder: **Comte, Pierre-André, Dr.,
Seltisbergerstrasse 19, CH-4410 Liestal (CH)**

(74) Vertreter: **Eder, Carl E., Patentanwaltsbüro EDER
AG Münchensteinerstrasse 2, CH-4052 Basel (CH)**

2

## Beschreibung

Die Erfindung betrifft eine Leitvorrichtung gemäss dem Oberbegriff des Anspruchs 1.

Die Leitvorrichtung ist für Anwendungen bestimmt, bei denen die Leitvorrichtung zusätzlich zu einem möglichst grossen elektrischen Leitwert auch eine gute Deformierbarkeit, insbesondere Biegbarkeit, und eine grosse Ermüdungsbeständigkeit besitzen soll. Mit dem Begriff "Ermüdungsbeständigkeit" ist dabei und im folgenden die Beständigkeit gegen wechselnde Deformationen, wie Dehnungen und vor allem Biegungen sowie Torsionen gemeint.

Die Leitvorrichtung ist insbesondere dazu vorgesehen, vollständig oder mindestens teilweise in einen menschlichen oder tierischen Körper eingesetzt zu werden und einen Teil einer Stimulations- und/oder Messeinrichtung zu bilden. Die Leitvorrichtung ist beispielsweise zur Herzstimulation, d.h. zur Verwendung als Leitung und/oder Elektrode für einen Herzschrittmacher vorgesehen. Sie kann aber auch für andere Arten von Neuro- oder Muskel- oder Knochenstimulation verwendet werden. Ferner kann die Leitvorrichtung auch dazu dienen, um von Körper-Zellen erzeugte, elektrische Signale einer Messvorrichtung zuzuführen.

Die Leitvorrichtung kann also als Leitung dienen, um einen elektrischen Strom von einer Stromquelle zu einem in einem Körperteil angeordneten Aktivator oder von einem in einem Körperteil angeordneten Sensor zu einer Messvorrichtung zu leiten. Dabei kann der Aktivator bzw. Sensor durch ein separates, an das eine Ende der Leitvorrichtung angeschlossenes Organ oder durch das blanke Ende des Leiters bzw. der Leiter der Leitvorrichtung gebildet sein. Eventuell kann auch ein Längsabschnitt der Wendel oder die ganze Wendel der Leitvorrichtung blank sein und als Aktivator oder Sensor dienen. Mindestens im Fall, dass mindestens ein Teil des Leiters bzw. der Leiter als Aktivator oder Sensor dient, bildet die Leitvorrichtung eine Elektrode.

Aus der US-Patentschrift 3 572 344 ist beispielsweise eine Leitvorrichtung mit einer Leitung bekannt, die einen aus Isoliermaterial bestehenden Dorn aufweist, um den wendelartig mehrere Adern herumgewickelt sind, die ihrerseits je aus einem Kern aus Isoliermaterial und einem um diesem herumgewickelten Leiter bestehen. Die verschiedenen Leiter sind am einen Ende der Elektrode alle mit einem abgewinkelten Kontakt und am andern Leitungsende alle mit einer zum Verbinden mit einem Gerät bestimmten Stift verbunden.

Ferner sind aus der US-Patentschrift 3 596 662 für Herzschrittmacher vorgesehene Leitvorrichtungen mit einem zweiadrigen Kabel und zwei seitlich vorstehenden, stiftförmigen Kontakten bekannt. Das Kabel weist zwei nebeneinander verlaufende Drahtwendeln auf, die von einem Isoliermantel umschlossen sind und auch in ihrem Innern mit Isoliermaterial gefüllt sind.

Diese bekannten Leitvorrichtungen, bei denen die Wendel einen Kern aus Isoliermaterial enthält, sind jedoch relativ schlecht bieg- und dehnbar und haben nur eine geringe Ermüdungsbeständigkeit sowie relativ grosse Querschnittabmessungen. Zudem besitzen diese Leitvorrichtungen im Vergleich zu ihren Biegesteifheiten, Ermüdungsbeständigkeiten und Querschnittabmessungen nur einen verhältnismässig geringen elektrischen Leitwert.

In der medizinischen Praxis werden für Herzschrittmacher derzeit vorwiegend Leitvorrichtungen verwendet, die eine elektrisch leitende Wendel oder zwei zueinander koaxiale Wendeln aufweisen, wobei die Wendel bzw die äussere Wendel mit einem isolierenden Mantel versehen ist und innerhalb der bzw. der innern Wendel ein freier Hohlraum vorhanden ist. Jede Wendel ist dabei aus einem einzigen Draht oder aus in der Art eines mehrgängigen Gewindes nebeneinander verlaufenden Drähten gebildet. Diese bekannten Leitvorrichtungen weisen beispielsweise aus Kobaltlegierungen oder rostfreiem Stahl bestehende Drähte auf. Trotz der verhältnismässig grossen Festigkeit dieser Materialien treten aber bei diesen Leitvorrichtungen noch verhältnismässig häufig Brüche auf, und zwar vor allem Ermüdungsbrüche beim Biegen mit kleinen Krümmungsradien. Zudem besitzen diese Materialien nur eine relativ geringe, elektrische Leitfähigkeit. Wenn eine Wendel daher nur aus einem einzigen Leiter gebildet ist, hat sie einen verhältnismässig grossen, elektrischen Widerstand. Die Wendeln dieser bekannten Leitvorrichtung wurden daher meistens aus mehreren, in der Art eines mehrgängigen Gewindes nebeneinander verlaufenden Leitern gebildet, um dadurch den elektrischen Widerstand zu verkleinern, bzw. den Leitwert zu vergrössern. Wenn man jedoch, bei gleichbleibendem Leiter- und Wendeldurchmesser die Anzahl der Leiter vergrössert, aus denen die Wendeln besteht, wird die Steigung der Wendel grösser und damit die Ermüdungsbeständigkeit noch kleiner. Es sei hiezu beispielsweise auf die Publikation "Fatigue performances of stimulating electrodes" von P. Comte, E. Gysin und Th. Baehny, in Biomedizinische Technik, Band 28, Ergänzungsband, Mai 1983 verwiesen. Ferner enthält die Publikation "Failure of pacemaker electrode leads" von T. K. Kaul, G. D. Green und W. H. Bain, im European Journal of Cardiology, 1979, 10/5, Seiten 385 bis 394, Untersuchungsergebnisse über die Lebensdauer verschiedener bekannter Leitvorrichtungen von Herzschrittmachern. Gemäss dieser letzteren Publikation brechen in Patienten eingesetzte Elektroden mit einer Rate von 1 bis 7,3 % pro Jahr. Da ein solcher Bruch unter Umständen den Tod des betroffenen Patienten verursachen kann, sind Bruch-Raten in dieser Grösse natürlich ein schwerwiegender Nachteil.

Es sind auch bereits Leitvorrichtungen bekannt, deren Wendeln aus beispielsweise drei nebeneinander verlaufenden Leitern gebildet sind, von denen jeder aus einem Verbund-Draht gebildet ist, der aus Silber und der unter dem Handelsnamen MP 35 N bekannten Kobalt-Nickellegierung besteht. Der Mantel des Drahtes ist dabei aus sechs aus der genannten Kobalt-Nickellegierung bestehenden Segmenten gebildet, die einen Kern aus Silber umschliessen und fest mit diesem und miteinander verbunden sind. Es sei hiezu beispielsweise auf die Publikation "Neue Schrittmachersonden - ein Bericht aus Montreal" von H. J. Bisping, in Biomedizinische Technik, Band 25, 1980, Seiten 170 bis 175 verwiesen.

Untersuchungen an Leitvorrichtungen mit aus derartigen Verbund-Drähten gebildeten Wendeln haben jedoch gezeigt, dass diese Wendeln schon durch verhältnismässig geringe Deformationen plastisch verformt werden. Beim Implantieren einer derartigen Leitvorrichtung in den Körper eines Patienten können daher bleibende Bögen oder sogar knickartige Verformungen entstehen. Bei derart plastisch verformten Stellen ist die Ermüdungsbeständigkeit dann beträchtlich reduziert.

Die US-Patentschrift 3 333 045 offenbart ein zweiadriges Kabel, das einenends mit einem Herzschrittmacher verbunden ist und andernends blanke, in ein Herz hineinragende Ader-Enden hat. Jede Ader des Kabels weist ein wendelförmiges Leiterbündel mit sieben miteinander verseilten Leitern auf, die je aus sieben miteinander verdrillten Drähten bestehen. Jeder der Drähte besteht aus einer zentralen Silberfaser und sechs darum herum angeordneten Fasern aus rostfreiem Stahl, wobei die sieben Fasern miteinander verlötet sind.

Dieses bekannte Kabel ist also aus Verbund-Drähten gebildet, gleich wie die Verbund-Drähte gemäss der vorgängig kommentierten Publikation "Neue Schrittmachersonden - ein Bericht aus Montréal" von H. J. Bisping. Die aus miteinander verdrillten Drähten bestehenden Leiter des Kabels gemäss der US-Patentschrift 3 333 045 sind zu einem Leiterbündel verseilt. Die Drähte haben einen Durchmesser von 0.0018 Zoll, d.h. 0.046 mm. Der Durchmesser eines aus sieben verdrillten Leitern gebildeten Leiters liegt daher in der Grösse von 0,15 mm. Der Durchmesser eines aus sieben miteinander verseilten Leitern gebildeten Leiterbündels beträgt dann etwa 0,45 mm.

Der Erfindung liegt nun die Aufgabe zugrunde, eine Leitvorrichtung zu schaffen, die, bei möglichst kleinem Aussendurchmesser der Wendel eine grosse Ermüdungsbeständigkeit und einen verhältnismässig grossen elektrischen Leitwert bzw. kleinen elektrischen Widerstand aufweist.

Diese Aufgabe wird durch eine Leitvorrichtung gelöst, die nach der Erfindung gemäss dem Anspruch 1 ausgebildet ist. Vorteilhafte Ausgestaltungen der Erfindung gehen aus den abhängigen Ansprüchen hervor.

Die erfindungsgemässe Leitvorrichtung weist zumindest eine Wendel auf, die von Leitern gebildet ist. Beispielsweise kann die bzw. jede Wendel mindestens zwei in der Art eines mehrgängigen Gewindes nebeneinander verlaufende Leiter aufweisen, wobei vorzugsweise höchstens vier oder fünf Leiter pro Wendel vorhanden sind.

Der bzw. jeder Leiter besteht aus mindestens zwei zusammengehaltenen, zusammen ein Bündel bildenden Drähten, wobei zweckmässigerweise mindestens drei, vorzugsweise mindestens fünf und beispielsweise mindestens sieben Drähte pro Leiter vorhanden sind. Bei einer vorteilhaften Ausführungsform ist der bzw. jeder Leiter blank, wobei aber die Wendel mindestens in einem Teilbereich ihrer Länge mit einem elektrisch isolierenden Mantel umhüllt sein kann. Im übrigen kann die Leitvorrichtung nur eine Wendel oder zwei oder eventuell sogar mehr als zwei Wendeln aufweisen, die je aus Leitern gebildet und gegeneinander isoliert sind, wobei die Wendeln vorzugsweise koaxial zueinander angeordnet sind.

Der Erfindungsgegenstand soll nun anhand in der Zeichnung dargestellter Ausführungsbeispiele erläutert werden. In der Zeichnung zeigt

die Figur 1 einen Längsabschnitt einer Leitvorrichtung, wobei der Mantel aufgeschnitten und die Wendel in Ansicht gezeichnet ist,

die Figur 2 einen Querschnitt durch einen Leiter, in grösserem Massstab,

die Figur 3 eine Ansicht eines einzelnen, ungewickelten, d.h. geradlinig verlaufenden Leiters,

die Figur 4 ein Diagramm zur Veranschaulichung der Abhängigkeit des Widerstandes pro Längeneinheit der Wendel von der Anzahl Leiter, die Figur 5 eine schematische Ansicht einer Prüfvorrichtung für die Untersuchung der Ermüdungsbeständigkeit,

die Figur 6 ein Diagramm zur Veranschaulichung des Zusammenhangs zwischen Krümmungsradius und Ermüdungsbeständigkeit der Wendeln,

die Figur 7 eine Variante einer Wendel und

die Figur 8 einen schematisierten Längsschnitt durch eine Variante einer Leitvorrichtung mit zwei Wendeln.

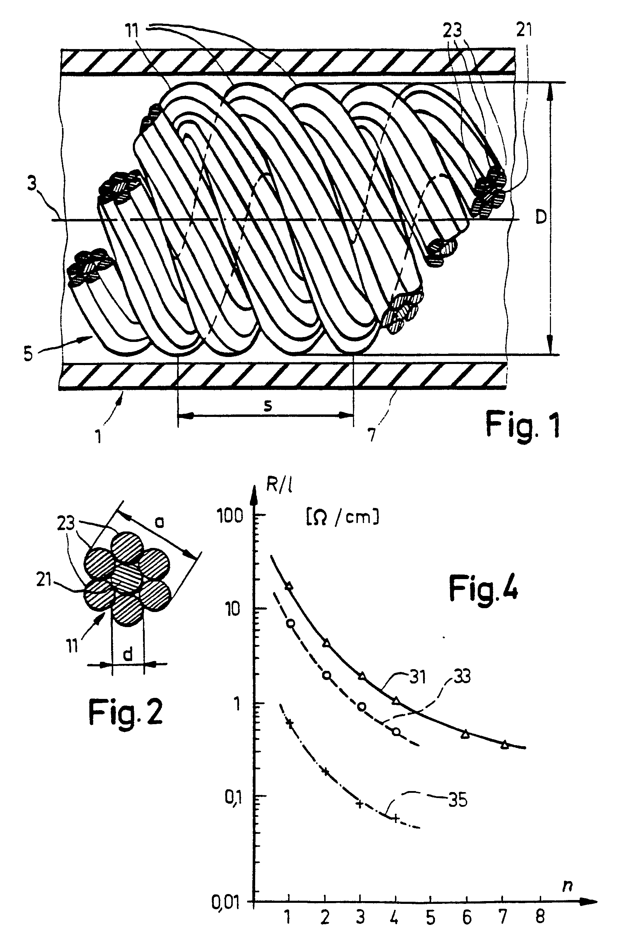

Die längliche, biegbare, elektrische Leitvorrichtung 1, von der in der Figur 1 ein Längsabschnitt dargestellt ist, weist eine elektrisch leitende, um eine Längsachse 3 herum verlaufende Wendel 5 und einen diese satt oder mit etwas Spiel umhüllenden, elektrisch isolierenden Mantel 7 auf. Der Mantel 7 besteht aus einem elektrisch isolierenden, gummielastischen, gut biegbaren und biokompatiblen Material, beispielsweise einem Elastomer auf Polyurethan oder Siliconbasis. Die Wendel 5 weist n Leiter 11, nämlich mindestens zwei sowie zweckmässigerweise höchstens fünf und vorzugsweise höchstens vier Leiter 11 auf. Die zur Wendel gehörenden Leiter 11 bilden zusammen eine einlagige Wicklung und verlaufen in der Art eines mehrgängigen Gewindes nebeneinander, so dass die Windungen der Leiter 11, bei undeformierter Leitvorrichtung 1, einander in axialer Projektion überdecken und zusammen einen Zylindermantel aufspannen. Bei dem in der Figur 1 dargestellten Ausführungsbeispiel der Leitvorrichtung sind als Beispiel drei Leiter 11 gezeichnet, die in der Art eines dreigängigen Gewindes nebeneinander verlaufen. Der Aussendurchmesser der Wendel 5, oder genauer gesagt, der Durchmesser von ihrer in undeformiertem Zustand kreiszylindrischen Hüllfläche, ist mit D bezeichnet. Der von den Windungen der Leiter 11 umschlossene, zentrale Innenbereich der Wendel 5 bildet einen freien, offenen Hohlraum.

Jeder Leiter 11 besteht aus mindestens zwei, zweckmässigerweise mindestens drei und vorzugsweise mindestens fünf und höchstens etwa 20 Drähten. Bei dem in der Figur 1 dargestellten Ausführungsbeispiel weist jeder Leiter 11, wie besonders deutlich aus der Figur 2 ersehen werden kann, sieben Drähte 21, 23 auf. Der Draht 21 bildet die Seele des Leiters 11. Die sechs andern, äussern Drähte sind gleichmässig um den Draht 21 herum verteilt. Die Drähte 21, 23 sind im Querschnitt kreisförmig und haben den Durchmesser d. Dabei haben vorzugsweise mindestens alle um die Seele herum verteilten Drähte 23 oder überhaupt alle Drähte 21, 23 den gleichen Durchmesser, so dass jeder äussere Leiter 23 den zentralen Leiter 21 sowie zwei andere, äussere Leiter 23 berührt. Ein derart aus sieben Drähten gebildeter Leiter 11 hat im Querschnitt bezüglich seiner Mittelachse eine verhältnismässig gleichmässige Materialverteilung und eine verhältnismässig rundliche Umrissform. Die grösste Querschnittsabmessung, d. h. der Hüllkreisdurchmesser eines Leiters 11 ist mit a bezeichnet. Die Drähte 23 sind um die Längsachse des Leiters 11 und den Draht 21 herum geschlagen oder gedreht. Die Schlagrichtung bzw. der Drehsinn der Drähte 23 ist dabei entgegengesetzt zur Drehrichtung, mit dem die Leiter 11 um die Achse 3 herum verlaufen, könnte aber eventuell auch gleichsinnig mit der Drehrichtung der Leiter sein.

Die Steigung der Wendel 5, d. h. die Ganghöhe s der Leiter 11 ist mindestens ungefähr gleich dem Produkt Anzahl nebeneinander verlaufender Leiter 11 mal Hüllkreisdurchmesser der Leiter 11, d. h. mindestens ungefähr gleich dem Produkt $n \cdot a$, und für das in der Figur 1 dargestellte Ausführungsbeispiel also mindestens ungefähr gleich dem Produkt $3 \cdot a$. Die Ganghöhe s der Leiter 11 ist zweckmässigerweise höchstens 100 % grösser und vorzugsweise höchstens etwa 30 % grösser als das Produkt $n \cdot a$. Die Leiter 11 sind beispielsweise gemäss der Figur 1 derart verteilt und gewickelt, dass der Abstand zwischen benachbarten Windungen der Leiter 11 überall mindestens annähernd gleich gross ist. Die benachbarten Windungen der Leiter 11 können aneinander anliegen oder durch einen Zwischenraum getrennt sein, dessen axial gemessene Ausdehnung zweckmässigerweise höchstens ungefähr gleich dem Hüllkreisdurchmesser a eines Leiters 11 ist, und vorteilhafterweise höchstens ungefähr 30 % des Hüllkreisdurchmessers a des Leiters 11 beträgt.

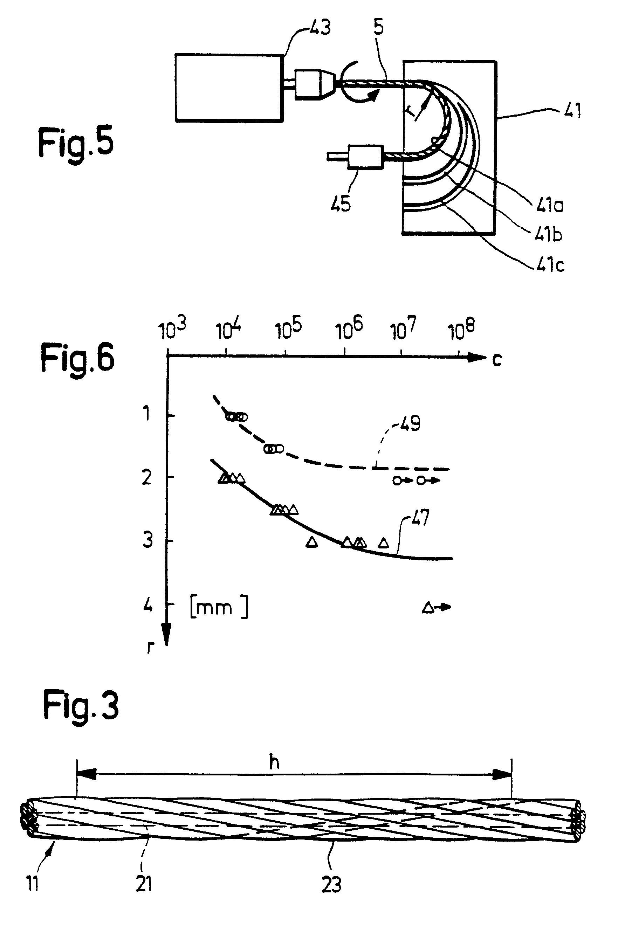

Die Schlaglänge eines Leiters 11, d. h. die Ganghöhe der geschlagenen bzw. zusammengedrehten, äusseren Drähte 23 ist in einen einzelnen, gerade verlaufenden Leiter 11 zeigenden Figur 3 mit h bezeichnet und die Anzahl geschlagener Drähte 23 eines Leiters sei mit m bezeichnet, wobei also m für den in den Figuren 1 bis 3 dargestellten Leiter 11 gleich sechs ist. Die Schlaglänge h ist grösser als das Produkt Anzahl geschlagene Drähte 23 mal Drahtdurchmesser, d. h. als das Produkt $m \cdot d$ und beträgt zweckmässigerweise mindestens das Zweifache und vorzugsweise mindestens das Dreifache des Produkts $m \cdot d$. Die Schlaglänge h kann dabei etwa das Zwanzig- bis Achzigfache des Drahtdurchmessers d betragen. Die Schlaglänge h beträgt zudem mindestens 25 % und vorzugsweise mindestens 40 % sowie höchstens 200 % und vorzugsweise höchstens 100 % der Länge einer Windung des Leiters 11. Die Schlaglänge h kann beispielsweise etwa 50 % bis 80 % der Länge einer Windung des Leiters 11 betragen. Zur Klarstellung sei noch bemerkt, dass hierbei mit der Länge einer Windung des Leiters 11, die mittlere, abgewikkelte Länge einer solchen Windung gemeint ist.

Dadurch, dass die Drähte 23 um die Achse eines Leiters 11 und den dessen Seele bildenden Draht 21 herum geschlagen bzw. gedreht sind, werden sie zu einem Bündel, d. h. einem Seil oder Zwirn verbunden. Die zum gleichen Leiter 11 gehörenden Drähte 21, 23 werden also zusammen gehalten, wobei aber die verschiedenen Drähte 21, 23 nicht starr miteinander verbunden sind, sondern nur aneinander anliegen und sich, zumindest innerhalb gewisser Grenzen, bezüglich einander bewegen können.

Wenn die Leitvorrichtung 1 für die Verwendung als Teil eines Herzschrittmachers bestimmt ist, kann an ihrem herzseitigen Ende als Aktivator eine Kontakt-Elektrode angeordnet sein, die den oder die Leiter 11 mit dem zu stimulierenden Gewebe verbindet. Die Kontakt-Elektrode ist dabei vorzugsweise mit einem den Innenraum des Mantels 7 und damit auch den von der Wendel 5 umschlossenen Hohlraum dicht abschliessenden Abschlussteil, beispielsweise einer den Endabschnitt des Mantels 7 unschliessenden, und an diesem befestigten Hülse, versehen. Am andern Ende weist die Leitvorrichtung einen Anschluss auf, der an einen elektrischen Impulsgeber anschliessbar ist. Für die möglichen Ausbildungen der Kontakt-Elektrode und des Anschlusses sei beispielsweise auf die zitierte Literatur und den sonstigen Stand der Technik verwiesen. Bei eingesetztem Herzschrittmacher verbindet die Wendel 5 den Impulsgeber elektrisch leitend mit der Kontakt-Elektrode. Die Leitvorrichtung 1 ist im übrigen derart ausgebildet, dass von ihrem an den Impulsgeber anschliessbaren Ende her zum Einführen der Leitvorrichtung 1 in einen Patienten vorübergehend ein sogenanntes Stilett, das durch einen drahtförmigen Dorn gebildet ist oder einen solchen aufweist, in den von der Wendel 5 begrenzten Hohlraum eingeschoben werden kann. Nach dem Entfernen des Stiletts kann der Hohlraum der Wendel eventuell auch an seinem zum Anschliessen an den Impulsgeber bestimmten Ende durch einen Abschlussteil abgeschlossen werden.

Die Drähte 21, 23 sind normalerweise durch den Mantel 7 und den dessen Innenraum mindestens am herzseitigen Ende der Leitvorrichtung 1 abschliessenden Abschlussteil gegen eine Berührung mit dem Blut und sonstigen Flüssigkeiten sowie festen Teilen des Körpers des Patienten, in den die Leitvorrichtung eingesetzt ist, geschützt. Da aber die Leiter 11 etwa bei einer Beschädigung des Mantels 7 unter Umständen doch mit Blut oder sonstigen Zellen und Geweben des Körpers des Patienten in Berührung kommen könnten, bestehen die die Leiter 11 bildenden Drähte zweckmässigerweise aus mindestens einigermassen biokompatiblen Materialien.

Der Wendel-Aussendurchmesser D kann beispielsweise etwa 0,5 mm bis 3 mm betragen. Mindestens im Fall, dass die Leitvorrichtung 1 zu einem Herzschrittmacher gehört, beträgt der Wendel-Aussendurchmesser D zweckmässigerweise höchstens 1,5 mm und vorzugsweise höchstens 1 mm. Der Durchmesser d der Drähte 21, 23 beträgt zweckmässigerweise höchstens 10 % und vorzugsweise höchstens 7 % des Aussendurchmessers D der Wendel 5. Der Drahtdurchmesser d beträgt ferner zweckmässigerweise höchstens 0,08 mm, vorzugsweise höchstens 0,06 mm und beispielsweise mindestens 0,02 mm bis 0,06 mm.

Die zu einem Leiter 11 gehörenden Drähte 21, 23 können entweder alle aus dem gleichen, elektrisch leitenden Material oder aus verschiedenen Materialien bestehen. Mindestens ein Teil der Drähte eines Leiters 11 soll aus einem Material bestehen, das vor allem gute mechanische Eigenschaften, wie eine grosse Elastizitätsgrenze, eine grosse Bruchfestigkeit und besonders eine grosse Dauerschwingfestigkeit hat. Dementsprechend sollen die aus diesem Material bestehenden Drähte gut elastisch deformierbar, und insbesondere mit kleinen Krümmungsradien biegbar sein, ohne dass Brüche entstehen.

Diesen Anforderungen genügende Materialen sind beispielsweise verschiedene kobalthaltige Legierungen, wie die unter dem Handelsnamen Syntacoben von Institut Straumann AG, Schweiz, gelieferte CoNiCrMoWFe-Legierung (Norm ASTM F 563-78), die unter dem Handelsnamen MP 35 N von The Standard Pressed Steel Co., USA, gelieferte CoNiCrMoTi-Legierung (Normen ISO 5832/6; ASTM F 562-78) und die unter dem Handelsnamen Elgiloy von Elgin National Watch Co., USA, gelieferte CoCrFeNi-Legierung. Des weitern können die Drähte oder ein Teil von ihnen statt aus diesen Kobaltlegierungen auch aus rostfreiem Stahl, wie etwa dem unter der Bezeichnung 316 L im Handel erhältlichen, Fe, Cr, Ni, Mo enthaltenden Stahl (Normen ISO 5832/1; ASTM F 138-76), oder eventuell aus Wolfram hergestellt werden.

Von diesen Materialien haben vor allem die Kobaltlegierungen und der rostfreie Stahl relativ niedrige elektrische Leitfähigkeiten. Beispielsweise liegen die elektrischen Leitfähigkeiten der erwähnten Kobaltlegierungen in der Grösse von $10^6$ S/m. Es ist nun aber ohne weiteres möglich, jeden Leiter aus Drähten zu bilden, die aus verschiedenen Materialien bestehen. Dabei kann jeder Leiter mindestens einen aus einem ersten Material mit hoher Bruchfestigkeit bestehenden Draht und mindestens einen aus einem zweiten Material mit hoher elektrischer Leitfähigkeit bestehenden Draht aufweisen. Das erste Material kann beispielsweise eine der genannten Kobaltlegierungen oder rostfreier Stahl sein. Das zweite Material kann beispielsweise Silber oder Kupfer sein, dessen elektrische Leitfähigkeit $60 \cdot 10^6$ S/m bzw. $57 \cdot 10^6$ S/m beträgt. Statt Silber oder Kupfer kommen auch noch Gold, Platin oder Aluminium als zweites Material in Frage. Das Material mit der höheren Leitfähigkeit kann dafür dann eine kleinere Bruchfestigkeit und Dauerschwingfestigkeit haben als das erste Material, d. h. die Kobaltlegierungen und rostfreier Stahl.

Wolfram liegt leitfähigkeitsmässig zwischen den Kobaltlegierungen oder rostfreiem Stahl und Silber oder Kupfer und kann eventuell je nach dem als erstes oder zweites Material verwendet werden. Dabei bestehen zweckmässigerweise mindestens die Hälfte, d. h. mindestens 50 %, und vorzugsweise mindestens 70 % der Drähte eines Leiters aus einem der ersten, eine hohe Festigkeit besitzenden Materialien, wobei es aber auch umgekehrt sein könnte.

Es wurden unter anderem Untersuchungen durchgeführt, bei denen für verschiedene Varianten von Wendeln der Widerstand pro Längeneinheit gemessen wurde. Einige der Untersuchungsergebnisse sind im in der Figur 4 dargestellten Diagramm veranschaulicht. In der Figur 4 ist auf der Abszisse die Variable n, d. h. die Anzahl der Leiter angegeben, aus denen eine Wendel gebildet ist. Auf der Ordinate ist der mit R/1 bezeichnete Widerstand pro Längeneinheit der Wendel in Ohm pro Zentimeter aufgetragen. Der Aussendurchmesser D der untersuchten Wendeln betrug 0,8 mm und die Wendeln waren alle möglichst dicht gewickelt, so dass benachbarte Windungen einander mindestens annähernd berührten.

Die Kurve 31 zeigt die Abhängigkeit des Widerstands pro Längeneinheit der Wendeln von der Anzahl Leiter für nicht erfindungsgemässe Leitvorrichtungen, bei denen der bzw. jeder Leiter aus einem einzigen, im Querschnitt kreisförmigen Draht mit einem Durchmesser von 0,1 mm gebildet ist, wobei der Draht aus der vorgängig erwähnten Kobaltlegierung MP 35 N besteht. Die Kurve 33 zeigt die Abhängigkeit des Widerstands pro Längeneinheit der Wendeln, die abgesehen von der Wendel mit nur einem einzigen Leiter, d. h. mit n = 1 erfindungsgemässe Leitvorrichtungen bildet, bei denen jeder Leiter aus sieben aus der Kobaltlegierung MP 35 N bestehenden, im Querschnitt kreisförmigen Drähten mit einem Durchmesser d von 0,05 mm gebildet ist. Die Kurve 35 betrifft mit Ausnahme des Puuktes n = 1 ebenfalls erfindungsgemässe Leitvorrichtungen, bei denen jeder Leiter aus sechs aus der Kobaltlegierung MP 35 N bestehenden Drähten mit einem Durchmesser d von 0,05 mm und einem Draht aus Kupfer mit einem Durchmesser d von 0,06 mm gebildet ist. Der Kupferdraht bildete dabei einen der äussern Drähte 23. Es sei hierbei bemerkt, dass für die Versuche ein Kupferdraht mit von den andern Drähten abweichenden Durchmesser verwendet wurde, weil kein Kupferdraht mit gleichem Durchmesser vorhanden war, und dass bei für die medizinische Anwendung vorgesehene Leitvorrichtungen zweckmässigerweise mindestens alle äussern Drähte 23 den gleichen Durchmesser haben.

Wie aus der Figur 4 ersichtlich ist, nimmt der Widerstand bei jeder der drei Arten von Leitern mit wachsender Leiter-Anzahl n ab. Bei jeweils gleicher Anzahl Leiter haben die Wendeln, deren Leiter aus sieben kobalthaltigen Drähten bestehen, einen kleineren Widerstand, als die Wendeln der nicht erfindungsgemässen Leitvorrichtungen, deren Leiter nur aus je einem Draht bestehen. Ferner haben die Wendeln, bei denen ein Draht der Leiter aus Kupfer besteht, einen wesentlich kleineren Widerstand als die Wendeln, deren Leiter ausschliesslich aus kobalthaltigen Drähten bestehen.

Selbstverständlich könnte der Kupferdraht statt einen der äusseren Drähte 23 auch den Draht 21, d. h. die Seele der Leiter bilden. Da die Seele kürzer ist als die um sie herum geschlagenen Drähte, würde der Widerstand dann bei gleichen Drahtdurchmessern noch etwas kleiner als bei den Wendeln gemäss der Kurve 35. Im übrigen könnte man auch mehr als einen Kupferdraht oder eben, wie vorgängig erwähnt, statt mindestens einen Kupferdraht mindestens einen Silber- oder eventuell Gold-, Platin-, aluminium- oder Wolframdraht vorsehen.

Es wurde auch die Ermüdungsbeständigkeit von Wendeln mit der schematisch in der Figur 5 dargestellten Prüfvorrichtung untersucht. Diese weist einen Block 41 oder auswechselbare Blöcke mit U-förmig verlaufenden Führungsnuten auf, von denen drei gezeichnet und mit 41a, 41b, 41c bezeichnet sind und mit denen die Wendeln wahlweise mit verschiedenen Krümmungsradien geführt werden können. Eine zu prüfende Wendel 5 wird in eine der Führungsnuten, beispielsweise die Führungsnut 41a eingelegt, so dass die Wendel durch den halbkreisförmigen Teil der Führungsnut 41a mit dem Krümmungsradius r gebogen wird. Eine Antriebsvorrichtung 43 weist einen Motor und ein um eine Drehachse drehbares Spannorgan auf. An diesem ist der eine aus der Führungsnut 41a herausragende Endabschnitt der Wendel 5 derart befestigt, dass seine Achse mit der Drehachse des Spannorganes zusammenfällt. Der andere Endabschnitt der Wendel 5 ragt ebenfalls aus der Führungsnut 41a heraus und ist mit einer Halte- und Lagervorrichtung 45 frei um seine Achse drehbar gehalten. Für die Versuche werden die Wendeln mit einer beispielsweise 3000 Umdrehungen pro Minute betragenden Drehzahl um ihre Achsen gedreht. Dadurch werden die Wendeln sowohl auf Biegung als auch auf Torsion beansprucht. Im übrigen sei auf die bereits zitierte Publikation "Fatigue performance of stimulating electrodes" von P. Comte et al. verwiesen, wo dieses Prüfverfahren ebenfalls erläutert ist.

Die Figur 6 enthält ein Diagramm, in dem einige Ergebnisse von Ermüdungsbeständigkeits-Prüfungen dargestellt sind, wobei auf der einen Achse der Krümmungsradius r der Wendeln in Millimeter und auf der andern Achse die mit c bezeichnete Anzahl Zyklen, d. h. Drehungen oder Biegungen der Wendeln aufgetragen ist. Die beiden Kurven 47, 49 zeigen die Messergebnisse für Wendeln, die je aus drei Leitern gebildet sind und einen Aussendurchmesser D von 0,8 mm haben. Dabei wurden die Versuche mit Wendeln ohne Mantel 7 durchgeführt. Die Kurve 47 betrifft Wendeln von nicht erfindungsgemässen Leitvorrichtungen, nämlich Wendeln, bei denen jeder der drei Leiter nur einen einzigen, aus der Kobaltlegierung MP 35 N bestehenden Draht mit einem Durchmesser d von 0,1 mm aufweist. Die Kurve 49 bezieht sich auf Wendeln von erfindungsgemässen Leitvorrichtungen, nämlich Wendeln, bei denen jeder der drei Leiter sieben aus der Kobaltlegierung MP 35 N bestehende Drähte mit einem Durchmesser d von 0,05 mm aufweist.

Bei den Untersuchungen wurde jeweils die Anzahl Zyklen ermittelt, bei der mindestens einer der aus einem einzigen Draht bestehenden Leiter der nicht erfindungsgemässen Leitvorrichtungen oder mindestens einer der äusseren Dräht 23 von mindestens einem Leiter 11 der erfindungsgemässen Leitvorrichtungen gebrochen ist. Die in den Kurven 47, 49 durch Kreise bzw. durch Dreiecke bezeichneten Messwerte bezeichnen die Zyklen-Zahlen, bei denen Brüche der genannten Art erfolgten. Diejenigen dieser Symbole, die einen Pfeil aufweisen, bezeichnen eine Anzahl von Zyklen, bei denen die Messung beendet wurde, ohne dass ein Bruch aufgetreten ist. Aus ähnlichen Langzeituntersuchungen der Ermüdungsbeständigkeit ist bekannt, dass ein Prüfling, der ungefähr $10^7$ Zyklen ohne Bruch übersteht, meistens auch bei einer wesentlich höheren Anzahl Zyklen nicht mehr bricht. Die mit Pfeilen versehenen Symbole deuten also an, dass die Wendeln bei den betreffenden Krümmungsradien praktisch nicht mehr brechen.

Der Krümmungsradius, oberhalb welchem die Wendeln $10^7$ Zyklen überleben und also praktisch unbegrenzte Lebensdauern haben, kann als kritischer Krümmungsradius bezeichnet werden. Die Figur 6 zeigt unter anderem, dass die Wendeln der erfindungsgemässen Leitvorrichtungen gemäss der Kurve 49 bereits bei Krümmungsradien von 2 mm an aufwärts eine in der Grössenordnung von $10^7$ liegende Anzahl Zyklen ohne Bruch überstehen und also einen zwischen 1,5 mm und 2 mm liegenden, kritischen Krümmungsradius haben. Dagegen brechen die Wendeln der nicht erfindungsgemässen Leitvorrichtungen gemäss der Kurve 47 bei einem Krümmungsradius vom 2 mm schon bei einer in der Grössenordnung von $10^4$ liegenden Anzahl Zyklen und haben einen oberhalb von 3 mm liegenden kritischen Krümmungsradius.

Dabei ist noch zu beachten, dass bei den Wendeln der nicht erfindungsgemässen Leitvorrichtung betreffenden Kurve 47 ein Messwert dem Bruch von mindestens einem der drei durch einen einzigen Draht gebildeten Leiter entspricht, wobei meistens alle drei Leiter an der gleichen Stelle der Wendeln gebrochen waren. Bei der Wendeln von erfindungsgemässen Leitvorrichtungen betreffenden Kurve 49 entspricht ein Messwert hingegen meistens dem Bruch von nur einem einzigen der sieben Drähte von einem der drei Leiter. Die Anzahl Zyklen bis zum vollständigen Bruch der Wendeln kann also bei den erfindungsgemässen Leitvorrichtungen noch wesentlich höher sein als die angegebenen Messwerte.

Ein wesentlicher Vorteil der untersuchten Wendeln der erfindungsgemässen Leitvorrichtungen besteht darin, dass sie durch Biegungen bis herab zu sehr kleinen Krümmungsradien, beispielsweise Krümmungsradien in der Grösse von 1 mm, praktisch ausschliesslich elastisch deformiert werden. Wenn also etwa eine Wendel, was beispielsweise beim Implantieren geschehen kann, vorübergehend mit einem so kleinen Krümmungsradius gebogen wird, bleibt nach Wegfall der von aussen an der Wendel angreifenden, verformenden Kraft, keine bleibende Verbiegung zurück.

Des weitern sind die Wendeln auch in ihrer Längsrichtung gut elastisch dehnbar und unempfindlich gegen Torsion.

Da Silber und Kupfer und auch die andern weiter vorn genannten Materialien mit hoher Leitfähigkeit kleinere Bruchfestigkeiten und Dauerschwingfestigkeiten als die erwähnten Kobaltlegierungen und rostfreier Stahl haben, wurde auch noch untersucht, wie sich allfällige Brüche eines aus einem der Materialien mit hoher Leitfähigkeit bestehenden Drahtes auf den Gesamtwiderstand der Wendeln mit Leitern auswirken, deren Drähte aus verschiedenen Materialien bestehen. Für diese Untersuchungen wurde bei Wendeln, deren Leiter aus einer Kobaltlegierung bestehende Drähte und einen Kupferdraht aufweisen, der letztere an mehreren Stellen absichtlich durchtrennt. Dabei zeigte sich, dass dies praktisch keine Widerstandsvergrösserung verursachte, weil diese Unterbrüche durch die übrigen Drähte überbrückt werden. Was die Festigkeit und Elastizität der Wendeln anbelangt, so werden diese durch allfällige Brüche der Drähte mit grösserer Leitfähigkeit auch nicht wesentlich beeinträchtigt.

Aus den in den Figuren 4 und 6 dargestellten Diagrammen geht also hervor, dass Wendeln von erfindungsgemässen Leitvorrichtungen den Wendeln von nicht erfindungsgemässen Leitvorrichtungen bei gleichem Wendel-Aussendurchmesser und gleicher Anzahl Leiter sowohl in bezug auf den elektrischen Leitwert als auch in bezug auf die Ermüdungsbeständigkeit überlegen sind.

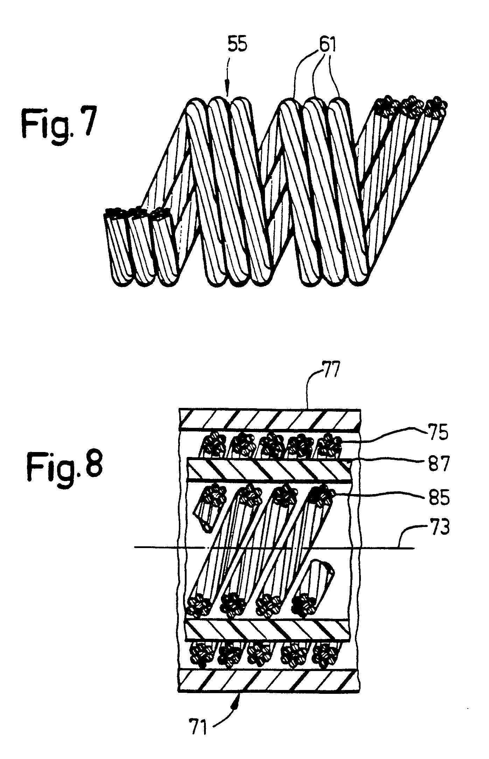

Die etwas vereinfacht in der Figur 7 dargestellte Wendel 55 weist nebeneinander verlaufende Leiter 61, beispielsweise drei Leiter 61 auf, von denen jeder aus beispielsweise sieben verseilten oder verzwirnten Drähten gebildet ist. Die Wendel 55 ist weitgehend ähnlich ausgebildet wie die in der Figur 1 ersichtliche Wendel 5, unterscheidet sich von dieser aber insbesondere dadurch, dass zwar der erste der drei Leiter am zweiten Leiter und dieser am dritten Leiter anliegt, dass aber zwischen einer Windung des dritten Leiters und der benachbarten Windung des ersten Leiters ein freier Zwischenraum vorhanden ist.

In der Figur 8 ist etwas schematisiert eine Leitvorrichtung 71 dargestellt, die eine Längsachse 73, eine äussere Wendel 75 und eine dazu koaxiale, innere Wendel 85 aufweist. Die beiden Wendeln 75, 85 können beispielsweise je drei Leiter aufweisen, von denen jeder aus sieben Drähten besteht, und auch sonst mehr oder weniger ähnlich ausgebildet sein wie die Wendel 5.

Die äussere Wendel 75 ist von einem dem Mantel 7 der Leitvorrichtung 1 entsprechenden, äusseren Mantel 77 umhüllt und zwischen den beiden Wendeln 75, 85 ist noch ein Mantel 87 vorhanden, der beispielsweise aus dem gleichen elektrisch isolierenden Material besteht, wie der äussere Mantel 77 und die beiden Wendeln elektrisch gegeneinander isoliert. Die beiden Wendeln 75, 85 können an ihren Enden mit separaten Kontakt-Elektroden bzw. Anschlüssen verbunden sein, so dass also die Leitvorrichtung 71 eine zweipolige Leitung bildet.

Bei den in den Figuren 1, 7, 8 der Zeichnung dargestellten Wendeln weist jeder Leiter eine von einem Draht gebildete Seele und sechs um diese und die Längsachse des Leiters herum geschlagene oder gedrehte Drähte auf. Selbstverständlich könnte man die Seele auch aus mehr als einem Draht bilden. Eventuell könnte man sogar nur einen einzigen Draht schraubenlinienartig um die mindestens einen Draht enthaltende Seele herumwickeln. Zweckmässigerweise sind jedoch mindestens zwei und vorzugsweise mindestens drei Drähte schraubenlinienartig um die Längsachse und allenfalls vorhandene Seele des bzw. jedes Leiters herum geschlagen bzw. gedreht. Die Leiter können jedoch auch ohne Seele hergestellt und ausschliesslich aus geschlagenen bzw. zusammengedrehten Drähten gebildet werden.

Die Drähte könnten statt durch Schlagen bzw. Drehen oder zusätzlich dazu auch dadurch verseilt werden, dass man mindestens einen Teil der Drähte eines Leiters miteinander verflechtet. Falls der Leiter eine unverflochtene Seele aufweist, werden mindestens zwei gegensinnig um die Seele herum verlaufende Drähte miteinander verflechtet. Vorzugsweise werden jedoch mindestens drei oder mindestens vier Drähte miteinander verflechtet. Selbstverständlich können auch alle Drähte eines Leiters verflochten sein. Wenn mindestens ein Teil der Drähte eines Leiters verflochten ist, gelten die weiter vorn für die Schlaglänge angegebenen Grössen und Verhältniswerte sinngemäss für die Flechtlänge eines Leiters, d. h. die Ganghöhe der verflochtenen Drähte.

Falls der Leiter eine Seele mit mindestens einem nicht geschlagenen bzw. nicht gedrehten oder nicht verflochtenen Draht und mindestens einen um die Seele herum geschlagenen oder um die Seele herum geflochtene Drähte aufweist, kann eventuell der geschlagene bzw. jeder der geschlagenen oder geflochtenen Drähte bandartig ausgebildet sein und statt eines runden Profils ein flaches Rechteckprofil aufweisen. Mindestens der bzw. jeder zur Seele gehörende Draht ist jedoch vorzugsweise im Querschnitt kreisförmig.

Des weitern wäre es möglich, jeden Leiter mit einem flexiblen Mantel zu versehen, der die zum Leiter gehörenden Drähte derart zusammenhält, dass sie mehr oder weniger aneinander anliegen, aber doch noch etwas bezüglich einander beweglich sind. Beim Vorhandensein eines derartigen Mantels müssten dann die Drähte nicht mehr unbedingt miteinander verseilt sein und könnten eventuell mehr oder weniger parallel zu einander verlaufen. Ein solcher Mantel könnte beispielsweise aus einem Elastomer bestehen.

Die Leitvorrichtungen können nicht nur als Teile von Herzschrittmachern, sondern auch als Teile von anderen Einrichtungen verwendet werden, bei denen im Innern eines menschlichen oder tierischen Körpers entweder irgendwelche Körperteile mit elektrischen Spannungen und/oder Strömen stimuliert oder vom Körper erzeugte, elektrische Spannungen und/oder Ströme erfasst werden sollen. Dabei können die Leitvorrichtungen entweder vollständig im Körper eingesetzt sein oder sich zum Teil innerhalb und zum Teil ausserhalb von diesem befinden. Im übrigen können die Wendeln wie bei den in den Figuren 1 und 8 dargestellten Ausführungsbeispielen mit einem isolierenden Mantel umhüllt sein. Es besteht aber auch die Möglichkeit, eine Wendel nur in einem Teil ihrer Länge mit einem isolierenden Mantel zu umhüllen oder überhaupt keinen solchen Mantel vorzusehen. In diesem Fall ist ein direkter, elektrisch leitender Kontakt zwischen der Wendel und dem menschlichen oder tierischen Körperteil, in den die Wendel eingesetzt ist, möglich, so dass also die Wendel eine eigentliche Elektrode, d. h. eine Kontakt-Elektrode bildet.

**Patentansprüche**

1. Leitvorrichtung zum mindestens teilweisen Einsetzen in einen menschlichen oder tierischen Körper, beispielsweise für einen Herzschrittmacher, mit mindestens einer aus elektrischen Leitern (11, 61) gebildeten Wendel (5, 55, 75, 85), wobei jeder Leiter (11) aus mindestens zwei zusammengehaltenen Drähten (21, 23) gebildet ist, dadurch gekennzeichnet, dass die zu einer gleichen Wendel (5, 55, 75, 85) gehörenden Leiter (11, 61) zusammen eine einlagige Wicklung in der Art eines mehrgängigen Gewindes bilden.

2. Leitvorrichtung nach Anspruch 1, dadurch gekennzeichnet, dass der Leiter (11) mindestens drei, vorzugsweise mindestens fünf und beispielsweise sieben Drähte (21, 23) aufweist.

3. Leitvorrichtung nach Anspruch 1 oder 2, dadurch gekennzeichnet, dass die zum gleichen Leiter (11) gehörenden Drähte (21, 23) nicht starr miteinander verbunden, sondern, zumindest innerhalb gewisser Grenzen, bezüglich einander beweglich sind, wobei die Drähte (21, 23) einander derart berühren, dass ein allfälliger Bruch eines Drahtes (21, 23) durch mindestens einen anderen Draht elektrisch überbrückt werden kann.

4. Leitvorrichtung nach einem der Ansprüche 1 bis 3, dadurch gekennzeichnet, dass der Leiter (11) seil- oder zwirnartig ausgebildet ist und/oder mindestens ein Teil der Drähte (21, 23) schraubenlinienförmig um die Achse des Leiters (11) herum gedreht und/oder miteinander verflochten ist, wobei mindestens ein Draht, zweckmässigerweise mindestens zwei, und vorzugsweise mindestens drei Drähte (23) um die Achse des Leiters (11) herum verläuft bzw. verlaufen.

5. Leitvorrichtung nach Anspruch 4, dadurch gekennzeichnet, dass der Leiter (11) mindestens einen eine Seele bildenden Draht (21) aufweist, der von zumindest einem Draht und vorzugsweise von zumindest drei Drähten (23) umschlossen ist.

6. Leitvorrichtung nach Anspruch 4 oder 5, dadurch gekennzeichnet, dass die Schlaglänge (h) bzw. Flechtlänge des Leiters (11) mindestens 25 % und vorzugsweise mindestens 40 % sowie höchstens 200 % der Länge einer Windung des Leiters (11) beträgt.

7. Leitvorrichtung nach einem der Ansprüche 1 bis 6, dadurch gekennzeichnet, dass die Drähte (21, 23) im Querschnitt kreisförmig sind und ihr Durchmesser (d) höchstens 10 % des Aussendurchmessers (D) der Wendel (5), zweckmässigerweise höchstens 0,08 mm und vorzugsweise höchstens 0,06 mm beträgt.

8. Leitvorrichtung nach einem der Ansprüche 1 bis 7, dadurch gekennzeichnet, dass jeder Leiter (11) aus zwei verschiedenen Materialien bestehende Drähte aufweist, von denen das erste eine grössere Bruchfestigkeit und das zweite eine grössere elektrische Leitfähigkeit besitzt als das jeweils andere, wobei das erste Material beispielsweise aus einer kobalthaltigen Legierung oder rostfreiem Stahl besteht und das zweite Material beispielsweise aus Silber, Kupfer, Aluminium, Gold oder Platin besteht.

9. Leitvorrichtung nach Anspruch 8, dadurch gekennzeichnet, dass mindestens die Hälfte, und vorzugsweise mindestens 70 % der Drähte eines Leiters (11) aus dem ersten Material besteht, wobei der Leiter (11) beispielsweise nur einen einzigen aus dem zweiten Material bestehenden Draht aufweist.

10. Leitvorrichtung nach einem der Ansprüche 1 bis 9, dadurch gekennzeichnet, dass die Wendel (5) bzw. die innerste Wendel (85) von zueinander koaxialen Wendeln (75, 85) in ihrem zentralen Innenteil einen freien Hohlraum aufweist, der mindestens vor dem Einführen der Leitvorrichtung in einen Körper an einem Ende offen ist, um das vorübergehende Einsetzen eines drahtförmigen Dorns zu ermöglichen.

11. Leitvorrichtung nach einem der Ansprüche 1 bis 10, dadurch gekennzeichnet, dass die einzelnen Leiter (11, 61) blank sind, dass neben einander angeordnete Windungen von Leitern (11, 61) einander zumindest teilweise berühren und dass die Wendel (5, 75, 85) mindestens in einem Teilbereich ihrer Länge von einem elektrisch isolierenden, gummielastischen, vorzugsweise aus einem Elastomer bestehenden Mantel (7, 77, 87) umhüllt ist.

12. Leitvorrichtung nach einem der Ansprüche 1 bis 11, gekennzeichnet durch zwei Wendeln (75, 85), von denen die eine (75) die andere (85) koaxial umschliesst und zwischen denen ein gummielastischer, elektrisch isolierender Mantel (87) vorhanden ist.

13. Leitvorrichtung nach einem der Ansprüche 1 bis 12, dadurch gekennzeichnet, dass jeder Draht (21, 23) über seinen ganzen Querschnitt aus einem einzigen Material besteht.

## Claims

1. Conductive device for at least partial insertion into a human or animal body, for example for a heart pacemaker, with at least one helix (5, 55, 75, 85) formed of electrical conductors (11, 61), wherein each conductor (11) is formed of at least two wires (21, 23) that are held together, characterised thereby, that the conductors (11, 61) belonging to the same helix (5, 55, 75, 85) together form a single-layer winding in the manner of a multiple thread.

2. Conductive device according to claim 1, characterised thereby, that the conductor (11) displays at least three, preferably at least five and for example seven wires (21, 23).

3. Conductive device according to claim 1 or 2, characterised thereby, that the wires (21, 23) belonging to the same conductor (11) are not rigidly connected one with the other, but at least within certain limits one movable with respect to the other, wherein the wires (21, 23) touch one the other in such a manner that a possible breakage one wire (21, 23) can be bridged over electrically by at least one other wire.

4. Conductive device according to one of the claims 1 to 3, characterised thereby, that the conductor (11) is constructed in the manner of a rope or yarn and/or at least a part of the wires (21, 23) is turned helically around the axis of the conductor (11) and/or plaited together, wherein at least one wire, expediently at least two and preferably at least three wires (23) extends or extend around the axis of the conductor (11).

5. Conductive device according to claim 4, characterised thereby, that the conductor (11) displays at least one wire (21), which forms a core and is enclosed by at least one wire and preferably at least three wires (23).

6. Conductive device according to claim 4 or 5, characterised thereby, that the lay (h) or pitch of plaiting of the conductor (11) amounts to at least 25 % and preferably at least 40 % as well as at most 200 % of the length of a turn of the conductor (11).

7. Conductive device according to one of the claims 1 to 6, characterised thereby, that the wires (21, 23) are circular in cross-section and their diameter (d) amounts to at most 10 % of the external diameter (D) of the helix (5), expediently at most 0.08 millimetres and preferably at most 0.06 millimetres.

8. Conductive device according to one of the claims 1 to 7, characterised thereby, that each conductor (11) displays wires consisting of two different materials, of which the first displays a greater breaking strength and the second a greater electrical conductivity than the respective other, wherein the first material consists for example of an alloy containing cobalt or of stainless steel and the second material consists for example of silver, copper, aluminium, gold or platinum.

9. Conductive device according to claim 8, characterised thereby, that at least half and preferably at least 70 % of the wires of a conductor (11) consist of the first material, wherein the conductor (11) for example displays only a single wire consisting of the second material.

10. Conductive device according to one of the claims 1 to 9, characterised thereby, that the helix (5) or the innermost helix (85) of mutually co-axial helices (75, 85) in its central interior part displays a free interior space which, at least before the introduction of the conductive device into a body, is open at one end in order to enable the temporary insertion of a wire-shaped thorn.

11. Conductive device according to one of the claims 1 to 10, characterised thereby, that the individual conductors (11, 61) are blank, that turns of conductors (11, 61) arranged one beside the other at least partly touch one the other and that the helix (5, 75, 85) is enveloped in at least a partial region of its length by an electrically insulating, rubber-elastic sheath (7, 77, 87) preferably consisting of an elastomer.

12. Conductive device according to one of the claims 1 to 11, characterised by two helices (75, 85), of which the one (75) encloses the other (85) co-axially and between which an electrically insulating, rubber-elastic sheath (87) is present.

13. Conductive device according to one of the claims 1 to 12, characterised thereby, that each wire (21, 23) consists of a single material across its entire cross-section.

## Revendications

1. Dispositif conducteur, pour insertion au moins partielle dans un corps humain ou animal, par exemple pour un stimulateur cardiaque, avec au moins une hélice (5, 55, 75, 85) formée par des conducteurs électriques (11, 61), chaque conducteur (11) étant formé par au moins deux fils maintenus ensemble (21, 23), caractérisé par le fait que les conducteurs (11, 61) appartenant à une même hélice (5, 55, 75, 85) forment ensemble un enroulement à une seule couche, à la manière d'un filetage à plusieurs filets.

2. Dispositif conducteur selon revendication 1, caractérisé par le fait que le conducteur (11) présente au moins trois fils, de préférence au moins cinq et par exemple sept fils (21, 23).

3. Dispositif conducteur selon revendication 1 ou 2, caractérisé par le fait que les fils (21, 23) appartenant au même conducteur (11) ne sont pas rigidement liés les uns aux autres, mais sont mobiles les uns par rapport aux autres, au moins à l'intérieur de certaines limites, les fils (21, 23) se touchant mutuellement de manière qu'une rupture éventuelle d'un fil (21, 23) puisse être shuntée électriquement par au moins un autre fil.

4. Dispositif conducteur selon l'une des revendications 1 à 3, caractérisé par le fait que le conducteur (11) est aménagé à la manière d'un câble ou d'un fil en torsade et/ou qu'au moins une partie des fils (21, 23) est enroulée en hélice autour de l'axe du conducteur (11), et/ou est tressée, au moins un fil, avantageusement au moins deux et de préférence au moins trois fils (23) suivant un tracé disposé autour de l'axe du conducteur (11).

5. Dispositif conducteur selon revendication 4, caractérisé par le fait que le conducteur (11) présente au moins un fil (21) constituant une âme, lequel est entouré par au moins un fil et de préférence par au moins trois fils (23).

6. Dispositif conducteur selon revendication 4 ou 5, caractérisé par le fait que le pas de câblage (h) ou le pas de tressage du conductcur (11) vaut au moins 25 % et de préférence au moins 40 %, et au maximum 200 %, de la longueur d'une spire du conducteur (11).

7. Dispositif conducteur selon l'une des revendications 1 à 6, caractérisé par le fait que les fils (21, 23) ont une section droite circulaire et par le fait que leur diamètre (d) vaut au maximum 10 % du diamètre extérieur (D) de l'hélice (5), avantageusement au maximum 0,08 mm et de préférence au maximum 0,06 mm.

8. Dispositif conducteur selon l'une des revendications 1 à 7, caractérisé par le fait que chacun des conducteurs (11) présente des fils constitués par deux matériaux différents, le premier possédant une plus grande résistance à la rupture et le deuxième une plus grande conductibilité électrique que l'autre, le premier matériau étant par exemple fait d'un alliage contenant du cobalt ou fait d'acier inoxydable, et le deuxième matériau étant constitué par exemple d'argent, cuivre, aluminium, or ou platine.

9. Dispositif conducteur selon revendication 8, caractérisé par le fait qu'au moins la moitié et de préférence au moins 70 % des fils d'un conducteur (11) sont faits du premier matériau, le conducteur (11) présentant par exemple seulement un fil fait du deuxième matériau.

10. Dispositif conducteur selon l'une des revendications 1 à 9, caractérisé par le fait que l'hélice (5), ou l'hélice la plus à l'intérieur (85) s'il y a plusieurs hélices coaxiales (75, 85), présente dans sa partie intérieure centrale une cavité libre qui, au moins avant l'introduction du dispositif conducteur dans un corps, est ouverte en une extrémité afin de permettre l'introduction provisoire d'un mandrin en forme de fil.

11. Dispositif conducteur selon l'une des revendications 1 à 10, caractérisé par le fait que les conducteuns individuels (11, 61) sont dénudés, par le fait que des spires de conducteurs (11, 61) agencées côte à côte se touchent les unes les autres, au moins partiellement, et par le fait que l'hélice (5, 75, 85) est enveloppée, au moins sur une région partielle de sa longueur, par une gaine (7, 77, 87) électriquement isolante, ayant l'élasticité d'un caoutchouc, de préférence faite en un élastomère.

12. Dispositif conducteur selon l'une des revendications 1 à 11, caractérisé par deux hélices (75, 85), dont l'une (75) entoure coaxialement l'autre (85), et entre lesquelles se trouve une gaine électriquement isolante (87), ayant l'élasticité d'un caoutchouc.

13. Dispositif conducteur selon l'une des revendications 1 à 12, caractérisé par le fait que chaque fil (21, 23) est constitué par un matériau unique sur toute sa section droite.

Fig. 1

Fig. 2

Fig. 4

Fig.5

Fig.6

Fig.3

Fig. 7

Fig. 8